# DEMANDE DE BREVET EUROPEEN

(11) **EP 4 523 676 A1**
(43) Date de publication de la demande: **19.03.2025**
(21) Numéro de dépôt: 24198084.6
(22) Date de dépôt: 03.09.2024
(51) Int. Cl.: A61K 8/9771, A61Q 1/10

(54) **EXTRAIT DE GINKGO BILOBA POUR STIMULER LA CROISSANCE DES CILS**

(30) Priorité: 13.09.2023 FR 2309617
(71) Demandeur: Chromavis France, 75116 Paris (FR)
(72) Inventeur: FRAISSE, Thibaut, 75116 PARIS (FR)
(74) Mandataire: Cabinet Le Guen Maillet

(57) **Abrégé**

La présente invention concerne l'utilisation cosmétique d'un extrait de ginkgo biloba comprenant un mélange de bilobalide et de ginkgolides A, B et C, ou d'une composition en comprenant, pour stimuler la croissance des cils ou améliorer l'apparence des cils.

## Description

La présente invention concerne un extrait de ginkgo biloba pour son utilisation cosmétique pour stimuler la croissance et la repousse des cils ou améliorer l'apparence des cils. L'invention concerne également une méthode pour stimuler la croissance et la repousse des cils ou améliorer l'apparence des cils ainsi qu'un kit pour la mise en oeuvre de cette méthode.

### ETAT DE LA TECHNIQUE

Les cils poussent sur les bords libres des paupières inférieures et supérieures. Ils présentent une longueur de 6 à 12 mm en moyenne. Le nombre de cils au niveau des paupières supérieures oscille entre 150 à 200 par paupière. Le nombre de cils au niveau des paupières inférieures oscille entre 50 à 150.

Les follicules pileux suivent un cycle qui leur est propre. On distingue la phase anagène de croissance qui dure environ trente jours, puis la phase intermédiaire catagène, une phase de repos qui stoppe la croissance et qui dure environ quinze jours, puis la phase télogène de fin de vie du cil qui laisse place au démarrage d'un cycle d'un nouveau cil.

Aujourd'hui l'apparence physique prend une place importante dans la société. Etant donné que les cils bordent les yeux, leur aspect, pigmentation, volume ou quantité est un critère esthétique important, notamment chez les femmes.

De nombreux facteurs sont susceptibles d'altérer l'aspect des cils, tels qu'un maquillage trop conséquent, l'utilisation de produits cosmétiques agressifs, les changements hormonaux, la ménopause, le stress ou un problème de santé. L'altération peut aller d'une apparence clairsemée à une perte totale des cils. La perte totale des cils est nommée hypo trichose.

A ce jour, l'approche la plus courante envisagée pour traiter ce problème est une approche thérapeutique.

A titre d'agents thérapeutiques connus pour stimuler la pousse et la croissance des cils on citera les produits utilisés dans le traitement des glaucomes.

Les analogues de prostaglandines (PGA) sont couramment utilisés dans le traitement du glaucome et agissent en réduisant la pression intraoculaire.

Cependant, l'un des effets secondaires le plus souvent observé chez les patients traités est la stimulation de la croissance des cils, la repousse, la pigmentation renforcée et l'augmentation du volume des cils.

Pour cette raison, les analogues de prostaglandines (PGA) sont aujourd'hui utilisés chez des personnes non atteintes du glaucome mais chez lesquelles la stimulation de la pousse et de la croissance des cils est désirée.

Le mécanisme d'action des analogues de prostaglandines dans le traitement du glaucome est complexe mais il est établi qu'ils agissent comme neurotransmetteurs en se liant à des récepteurs spécifiques de prostaglandines présents à la surface de la membrane cellulaire et de l'enveloppe nucléaire des follicules pileux (Simon K Law Clin Ophthalmol. 2010; 4: 349-358). Par l'intermédiaire de ces récepteurs, les analogues de prostaglandines sont capables d'activer une transition de la phase télogène à la phase anagène. A titre d'exemple, une étude confirme ainsi que le lanatoprost, un analogue de prostaglandines, est capable d'induire une hypertrichose, c'est-à-dire une croissance des cils, pour 77% de patients (Demitsu et al.). Une autre étude, non publiée, confirme également une hypertrichose chez des patients traités avec du bimatoprost, un autre analogue de prostaglandines.

Quelques produits à base d'analogues de prostaglandines ont été proposés sur le marché. On citera à titre d'exemple le produit commercialisé sous la marque Latisse^{®} par la société Allergan Inc. qui comprend 0,03% de bimatoprost. On citera également le produit commercialisé sous la marque Revitalash Advanced^{®} par la société Athena Cosmetics Inc. qui comprend 0,018 % de Dichloro Dihydroxy Difluoro Ethylcloprostenolamide (DDDE). Ces deux produits se présentent sous la forme d'eyeliners et s'appliquent au pinceau au raz des cils. La fréquence d'application recommandée est une fois par jour.

On citera également le médicament sous forme de collyre Xalatan qui comprend 0,005% de latanoprost.

Ces produits ne sont pas considérés comme des produits cosmétiques mais leur utilisation est souvent détournée pour la repousse des cils.

Bien qu'efficaces pour la repousse des cils, leur coloration, et l'augmentation de leur volume, les analogues de prostaglandines présentent des effets indésirables sur de nombreux utilisateurs. On relève notamment des allergies, des irritations, des inflammations, des pigmentations excessives de l'iris, et des sécheresses oculaires.

En outre, les analogues de prostaglandines demeurent des agents thérapeutiques et n'appartiennent pas à la catégorie des agents cosmétiques. Leur utilisation dans des produits cosmétiques est jusqu'à présent tolérée mais reste controversée. Ainsi, en Europe, le Comité Scientifique pour la Sécurité des Consommateurs a indiqué récemment dans une opinion que l'utilisation des analogues de prostaglandines en cosmétique pouvait présenter un risque pour la santé des consommateurs et qu'il n'existe pas suffisamment d'études pour déterminer la concentration maximale d'utilisation en cosmétique (SCCS/1635/21).

En dehors des analogues de prostaglandines, les antagonistes du récepteur aux minéralocorticoïdes (MRA) présentent également des effets secondaires sur la repousse des cils.

Ainsi, aujourd'hui, on ne dispose que d'ingrédients thérapeutiques pour stimuler la croissance des cils. Hormis le fait que leurs effets indésirables sont incompatibles avec une utilisation cosmétique, il convient de noter que ce sont des molécules synthétiques. Or, les consommateurs attendent de plus en plus qu'on leur propose des produits d'origine naturelle.

Il existe donc aujourd'hui le besoin d'identifier des agents actifs d'origine naturelle qui soient capables d'induire la croissance ou la repousse des cils, l'augmentation de leur longueur et épaisseur, le renforcement de leur pigmentation, et qui puissent être intégrés dans des produits cosmétiques et utilisés sans provoquer d'effets indésirables.

La société demanderesse a donc travaillé dans ce contexte afin de rechercher et d'identifier de tels composés d'origine naturelle capables de stimuler la croissance des cils.

Plus particulièrement, la société demanderesse a orienté ses recherches dans l'identification de neurotransmetteurs et plus spécifiquement dans la recherche de molécules naturelles antagonistes de récepteurs et présentant donc un mécanisme d'action proche de celui des analogues de prostaglandine et des antagonistes du récepteur aux minéralocorticoïdes.

### RESUME DE L'INVENTION

A cet effet, l'invention concerne l'utilisation cosmétique d'un extrait de ginkgo biloba comprenant un mélange de bilobalide et de ginkgolides A, B et C, ou d'une composition en comprenant, pour stimuler la croissance des cils ou améliorer l'apparence des cils.

Le ginkgo biloba comprend des agents actifs utilisés dans le domaine ophtalmique ou encore pour le traitement des désordres du système nerveux central (Fitoterapia Volume 71, Supplément 1, 1 August 2000, Pages 543-547).

A l'instar des antagonistes du récepteur aux minéralocorticoïdes (MRA) qui interagissent avec des récepteurs présents à la surface des cellules, certains terpènes lactones extraits de ginkgo biloba sont également des antagonistes de récepteurs, les récepteurs GABA_{A} et les récepteurs de glycine corticale selon une étude réalisée par Lidija Ivic et al. (THE JOURNAL OF BIOLOGICAL CHEMISTRY Vol. 278, No. 49, Issue of December 5, pp. 49279-49285, 2003). Les récepteurs de l'acide γ-aminobutyrique (GABA_{A}) et les récepteurs de glycine sont membres de la famille des canaux ioniques ligands dépendants qui inhibent la transmission synaptique dans le système nerveux central. Les ginkgolides et le bilobalide, des terpènes trilactones de ginkgo biloba, ont des effets variés sur le système nerveux central. Cette étude démontre que les ginkgolides sont des antagonistes des récepteurs à la glycine. Le bilobalide réduit également le courant ionique induit par la glycine, mais légèrement moins que les ginkgolides. Les ginkgolides et le bilobalide inhibent également les récepteurs GABA_{A}, avec un effet plus marqué pour le bilobalide.

Les ginkgolides et le bilobalide présentent des similarités structurelles : ils possèdent trois lactones et un groupe tert-butyl. Les ginkgolides A, B et C diffèrent par le nombre et la position des groupes hydroxyles. Ces terpènes lactones sont extraits des feuilles et racines du ginkgo biloba.

L'invention concerne aussi une méthode pour stimuler la croissance des cils ou améliorer l'apparence des cils comprenant l'application par voie topique sur la base des cils ou sur les bords libres des paupières supérieure ou inférieure d'une composition cosmétique comprenant un extrait de ginkgo biloba comprenant un mélange de bilobalide et de ginkgolides A, B et C.

L'invention concerne encore un kit cosmétique pour stimuler la croissance des cils ou améliorer l'apparence des cils comprenant une composition cosmétique comprenant un extrait de ginkgo biloba comprenant un mélange de bilobalide et de ginkgolides A, B et C et un système de délivrance comprenant un contenant et un applicateur en brosse de type mascara, en mine, ou en pinceau de type eyeliner.

### DEFINITIONS

Au sens de l'invention, une « *utilisation cosmétique* » s'entend d'une utilisation d'un extrait selon l'invention, ou d'une composition en comprenant, sur des sujets sains, ne souffrant pas d'une quelconque pathologie, en particulier d'une pathologie ophtalmique.

Au sens de l'invention, la « *stimulation de la croissance des cils ou l'amélioration de l'apparence des cils* » inclut l'augmentation de la longueur et de l'épaisseur des cils, l'augmentation du volume, l'apparition de nouveaux cils et le renforcement de leur pigmentation.

### DESCRIPTION DETAILLEE

L'invention concerne ainsi l'utilisation cosmétique d'un extrait de ginkgo biloba comprenant un mélange de bilobalide et de ginkgolides A, B et C, ou d'une composition en comprenant, pour stimuler la croissance des cils ou améliorer l'apparence des cils.

Selon une caractéristique de l'invention, la stimulation de la croissance et l'amélioration de l'apparence des cils sont choisies parmi l'augmentation de leur longueur et épaisseur, l'augmentation de leur pigmentation et/ou l'augmentation du nombre des cils.

Avantageusement, l'extrait comprend entre 2 et 4%, préférentiellement entre 2,5 et 3,5%, plus préférentiellement entre 2,6 et 3,2 %, encore plus préférentiellement 2,9% de bilobalide, et entre 2 et 4%, préférentiellement entre 2,5 et 3,5%, plus préférentiellement entre 2,8 et 3,4%, encore plus préférentiellement 2,9%, de ginkgolides A, B et C, en poids par rapport au poids total de l'extrait.

Un extrait de bilobalide et de ginkgolides A, B et C de ginkgo biloba utilisé dans le cadre de l'invention peut être obtenu par toute méthode connue de l'homme du métier à l'aide de solvants organiques ou d'eau. Des extraits disponibles dans le commerce peuvent être utilisés.

Selon un mode de réalisation de l'invention, l'extrait est contenu dans une composition cosmétique contenant un excipient cosmétiquement acceptable.

Selon un mode de réalisation de l'invention, la composition comprend entre 0,70 et 1,0 %, préférentiellement entre 0,75 et 0,96 %, plus préférentiellement entre 0,78 et 0,96 %, plus préférentiellement encore 0,87% de bilobalide, et préférentiellement entre 0,80 et 1,05 %, plus préférentiellement entre 0,84 et 1,02%, plus préférentiellement encore 0,87%, de ginkgolides A, B et C, en poids par rapport au poids total de la composition.

La composition cosmétique utilisée peut être pigmentée ou non.

Préférentiellement, lorsque la composition cosmétique doit être appliquée le soir avant le coucher, celle-ci est dépourvue de pigments.

Selon un mode de réalisation, la composition cosmétique utilisée comprend, de plus, au moins un autre composant choisi parmi l'huile de graine de ricin, l'huile de graine d'*Helianthus annuus*, l'huile de graine de *cannabis sativa*, le tocophérol, l'urée, le glutamate ou un de leurs mélanges.

Ces composants sont ajoutés dans la composition à une teneur comprise entre 0,01 et 10%, préférentiellement entre 0,01 et 0,5%, en poids par rapport au poids total de la composition.

D'autres composants classiquement utilisés dans le domaine cosmétique peuvent être ajoutés dans la composition tels que des conservateurs, des régulateurs de pH, des solvants, des huiles végétales ou d'autres agents actifs cosmétiques.

L'invention concerne encore une méthode pour stimuler la croissance ou améliorer l'apparence des cils, comprenant l'application par voie topique sur la base des cils ou sur les bords libres des paupières supérieures ou inférieures, d'un extrait, ou d'une composition cosmétique en comprenant, tels que décrits précédemment.

Avantageusement, l'application a lieu à l'aide d'un système de délivrance comprenant un contenant et un applicateur en brosse de type mascara, en mine ou en pinceau de type eyeliner.

L'application à l'aide d'un dispositif de type eyeliner est préférée dans le cadre de l'invention. En effet, le pinceau d'un eyeliner étant très fin, il permet une dépose uniforme de produit sur la ligne de pousse des cils.

Préférentiellement, l'application a lieu au moins une fois par jour, préférentiellement durant une période d'au moins un mois, plus préférentiellement d'au moins deux mois.

Avantageusement, l'application a lieu le soir au coucher, préférentiellement à l'aide d'un pinceau de type eyeliner.

L'application le soir permet de tenir compte du rythme circadien de la peau selon lequel les mécanismes de réparation ont lieu durant la nuit et de manière plus limitée le jour.

L'invention concerne enfin un kit cosmétique pour stimuler la croissance des cils ou améliorer l'apparence des cils comprenant un extrait, ou une composition cosmétique en comprenant, tels que décrits précédemment et un système de délivrance comprenant un contenant et un applicateur en brosse de type mascara, en mine ou en pinceau de type eyeliner.

Avantageusement, la composition est disposée à l'intérieur du contenant du kit.

### EXEMPLES

### Exemple 1 : Etude clinique

Une composition selon l'invention de type Eye liner non colorée et contenant du Bilobalide et des Gingkolides A, B, C à des concentrations de 0,87 %, respectivement, est préparée selon le tableau 1. Une composition témoin placébo, dépourvue de Bilobalide et de Gingkolides A, B, C, est préparée selon le tableau 2.

**[TABLE 1]**

| COMPOSITION SELON L'INVENTION | |
|---|---|
| Dénomination Technique | Intervalle % |
| Octyldodecanol | 15-25 |
| Excipient Ginkgo Biloba extrait sec sauf flavonoides, Bilobalide, Ginkgolide | 15-25 |
| Ricinus communis seed oil, | 10-17 |
| copernicia cerifera cera | 5-10 |
| flavonoides | 7,08 |
| cera microcristallina | 5-7 |
| C20-40 alcohols | 4-6 |
| cera alba | 3-5 |
| Helianthus Annus Seed oil | 3-5 |
| C10-18 triglycérides | 1-3 |
| methyl méthacrylate crosspolymer | 0,5-1,5 |
| Bilobalide | 0,87 |
| Ginkgolides A, B, C | 0,87 |
| glyceryl caprylate | 0,5-0,9 |
| Cannabis Sativa Seed Oil | 0,5-0,8 |
| ascorbyl palmitate | 0,2-0,5 |
| tridecyl trimellitate VP/hexadecene copolymer | 0,1-0,6 |
| citric acid | 0,01-0,10 |
| | |
| Total | qsp 100 % |

**[TABLE 2]**

| COMPOSITION TEMOIN PLACEBO | |
|---|---|
| Dénomination Technique | Intervalle % |
| Octyldodecanol | 20-35 |
| Ricinus communis seed oil, | 20-23 |
| copernicia cerifera cera | 10-13 |
| cera microcristallina | 7-9 |
| C20-40 alcohols | 6-8 |
| cera alba | 5-8 |
| Helianthus Annus Seed oil | 5-8 |
| C10-18 triglycérides | 2-5 |
| methyl méthacrylate crosspolymer | 0,9-1,5 |
| glyceryl caprylate | 0,7-1,4 |
| Cannabis Sativa Seed Oil | 0,3-0,8 |
| ascorbyl palmitate | 0,3-0,9 |
| tridecyl trimellitate VP/hexadecene copolymer | 0,1-0,6 |
| citric acid | 0,01-0,10 |
| | |
| Total | qsp 100 % |

Conditions de l'essai clinique :
Nombre de volontaires : 24 femmes âgées de 21 ans à 55 ans.
Nombre de volontaires retenues : 18 femmes après 9 semaines d'utilisation des compositions témoin placebo et des compositions selon l'invention (avec Bilobalide et Gingkolides A, B, C à 0,87 %) sur oeil droit et gauche avec une seule application par jour le soir entre 18h et 22h.
Conditions opératoires du banc optique : Les analyses de la longueur et de diamètre sont réalisées au moyen d'une caméra optique de haute résolution. Les images sont ensuite analysées à l'aide d'un logiciel adapté.
Analyse statistique : Le diamètre et la longueur des cils de la paupière supérieure sont évalués avec un logiciel adapté.

Résultats collectés entre 8 et 9 semaines d'utilisation

**[TABLE 3]**

| | TEMOIN PLACEBO | | INVENTION | |
|---|---|---|---|---|
| Volontaire paire | longueur, mm | diamètre, mm | longueur, mm | diamètre, mm |
| #1 | 10,36 | 0,07 | 12,27 | 0,1 |
| #2 | 9,67 | 0,07 | 11,67 | 0,1 |
| #3 | 10,36 | 0,08 | 11,88 | 0,1 |
| #4 | 9,76 | 0,07 | 13,27 | 0,1 |
| #5 | 9,76 | 0,06 | 13,76 | 0,09 |
| #6 | 11,24 | 0,08 | 12,52 | 0,09 |
| #7 | 10,39 | 0,07 | 12,67 | 0,1 |
| #8 | 10,42 | 0,07 | 11,85 | 0,09 |
| #9 | 10,33 | 0,07 | 13,21 | 0,09 |
| #10 | 9,97 | 0,07 | 13,79 | 0,1 |
| #11 | 10 | 0,07 | 11 | 0,09 |
| #12 | 11 | 0,07 | 12,76 | 0,1 |
| #13 | 10,21 | 0,08 | 13,03 | 0,1 |
| #14 | 10,85 | 0,07 | 14,21 | 0,1 |
| #15 | 10,15 | 0,08 | 13,21 | 0,09 |
| #16 | 10,39 | 0,08 | 12,33 | 0,09 |
| #17 | 10,61 | 0,08 | 11,91 | 0,09 |
| #18 | 10,42 | 0,08 | 12,24 | 0,1 |
| Moyenne | 10,3 | 0,074 | 12,6 | 0,0949 |
| Variance | 0,2 | 2,50E-05 | 0,7 | 5,90E-06 |
| Standard Déviation | 0,4 | 0,005 | 0,8 | 0,0024 |

Conclusion: Une utilisation entre 8 et 9 semaines de la composition selon l'invention contenant 0,87 % de bilobalide et 0,87% de gingkolide A, B, C induit de façon statistiquement significative une croissance des cils (Test de Student avec une probabilité p<0.05).

## Revendications

1. Utilisation cosmétique d'un extrait de ginkgo biloba comprenant un mélange de bilobalide et de ginkgolides A, B et C, ou d'une composition en comprenant, pour stimuler la croissance des cils ou améliorer l'apparence des cils.

2. Utilisation selon la revendication 1, dans laquelle l'extrait comprend entre 2 et 4%, préférentiellement entre 2,5 et 3,5%, plus préférentiellement entre 2,6 et 3,2 % de bilobalide, encore plus préférentiellement 2,9% et entre 2 et 4%, préférentiellement entre 2,5 et 3,5%, plus préférentiellement entre 2,8 et 3,4%, encore plus préférentiellement 2,9%, de ginkgolides A, B et C, en poids par rapport au poids total de l'extrait.

3. Utilisation selon l'une des revendications 1 à 2, dans laquelle l'extrait est contenu dans une composition cosmétique contenant un excipient cosmétiquement acceptable.

4. Utilisation selon la revendication 3, dans laquelle la composition cosmétique comprend entre 0,70 et 1,0 %, préférentiellement entre 0,75 et 0,96 %, plus préférentiellement entre 0,78 et 0,96 %, plus préférentiellement encore 0,87% de bilobalide, et préférentiellement entre 0,80 et 1,05 %, plus préférentiellement entre 0,84 et 1,02%, plus préférentiellement encore 0,87%, de ginkgolides A, B et C, en poids par rapport au poids total de la composition.

5. Utilisation selon l'une des revendications 3 à 4, dans laquelle la composition cosmétique est pigmentée ou non.

6. Utilisation selon l'une des revendications 3 à 5, dans laquelle la composition cosmétique comprend, de plus, au moins un autre composant choisi parmi l'huile de graine de ricin, l'huile de graine d'*Helianthus annuus*, l'huile de graine de cannabis sativa, le tocophérol, l'urée, le glutamate ou un de leurs mélanges.

7. Utilisation selon l'une des revendications précédentes, dans laquelle la stimulation de la croissance et l'amélioration de l'apparence des cils comprend l'augmentation de leur longueur et épaisseur, l'augmentation de leur pigmentation et/ou l'augmentation du nombre des cils.

8. Méthode pour stimuler la croissance des cils ou améliorer l'apparence des cils comprenant l'application par voie topique sur la base des cils ou sur les bords libres des paupières supérieure ou inférieure d'un extrait ou d'une composition cosmétique tels que décrits dans l'une des revendications 1 à 6.

9. Méthode selon la revendication 8, dans laquelle l'application a lieu à l'aide d'un système de délivrance comprenant un contenant et un applicateur en brosse de type mascara, en mine ou en pinceau de type eyeliner.

10. Méthode l'une des revendications 8 à 9, dans laquelle l'application a lieu au moins une fois par jour, préférentiellement durant une période d'au moins un mois, plus préférentiellement d'au moins deux mois.

11. Méthode selon l'une des revendications 8 à 10, dans laquelle l'application a lieu le soir au coucher, préférentiellement à l'aide d'un pinceau de type eyeliner, et dans laquelle la composition cosmétique est non pigmentée.

12. Composition cosmétique, **caractérisée en ce qu'**elle comprend entre 0,70 et 1,0 %, préférentiellement entre 0,75 et 0,96 %, plus préférentiellement, plus préférentiellement entre 0,78 et 0,96 %, plus préférentiellement encore 0,87% de bilobalide, et préférentiellement entre 0,80 et 1,05 %, plus préférentiellement entre 0,84 et 1,02%, plus préférentiellement encore 0,87%, de ginkgolides A, B et C, en poids par rapport au poids total de la composition.

13. Kit cosmétique pour stimuler la croissance des cils ou améliorer l'apparence des cils comprenant un extrait ou une composition cosmétique tels que décrits dans l'une des revendications 1 à 6 et 12, et un système de délivrance comprenant un contenant et un applicateur en brosse de type mascara, en mine, ou en pinceau de type eyeliner.

14. Kit selon la revendication 13, dans lequel la composition est disposée à l'intérieur du contenant.
